# EUROPEAN PATENT SPECIFICATION

(11) **EP 2 888 591 B1**
(45) Date of publication and mention of the grant of the patent: **29.08.2018**
(21) Application number: 13831222.8
(22) Date of filing: 21.08.2013
(51) Int. Cl.: G01N 33/53, G01N 21/64, G01N 21/75, G01N 33/569, G01N 33/58

(54) **METHOD FOR IMPROVED CELL IDENTIFICATION**
VERFAHREN ZUR VERBESSERTEN ZELLIDENTIFIKATION
PROCÉDÉ D'IDENTIFICATION AMÉLIORÉE DE CELLULES

(30) Priority: 21.08.2012 SE 1250936
(43) Date of publication of application: 01.07.2015
(73) Proprietor: Medetect AB, 223 81 Lund (SE)
(72) Inventor: ERJEFÄLT, Jonas, 247 31 Södra Sandby (SE)
(74) Representative: AWA Sweden AB
(86) International application number: PCT/SE2013/050984
(87) International publication number: WO 2014/031068

(56) References cited:
- WO-A2-2012/038068
- US-A1- 2011 195 413
- C K ANDERSSON ET AL: "Novel site-specific mast cell subpopulations in the human lung", THORAX, vol. 64, no. 4, 8 January 2009 (2009-01-08), pages 297-305, XP55187060, ISSN: 0040-6376, DOI: 10.1136/thx.2008.101683
- J. LECHAGO ET AL: "Simultaneous visualization of two antigens in the same tissue section by combining immunoperoxidase with immunofluorescence techniques.", JOURNAL OF HISTOCHEMISTRY AND CYTOCHEMISTRY, vol. 27, no. 9, 1 September 1979 (1979-09-01), pages 1221-1225, XP055246980, US ISSN: 0022-1554, DOI: 10.1177/27.9.113453
- PIRIS J ET AL: "An immunoperoxidase technique for the identification of gastrin producing cells", JOURNAL OF CLINICAL PATHOLOGY, BMJ PUBLISHING GROUP, GB, vol. 27, no. 10, 1 October 1974 (1974-10-01), pages 798-799, XP009111451, ISSN: 0021-9746, DOI: 10.1136/JCP.27.10.798
- Helga Semb 's Fund ET AL: "'Supported Comparison of Paired Immunofluorescence and Paired Immunoenzyme Staining Methods Based on Primary Antisera from the Same Species1", , 1 January 1982 (1982-01-01), pages 518-524, XP55246891, Retrieved from the Internet: URL:http://jhc.sagepub.com/content/30/6/51 8.full.pdf [retrieved on 2016-02-02]
- ANDERSSON C K ET AL.: 'Novel site-specific mast cell subpopulations in the human lung' THORAX vol. 64, 2009, pages 297 - 305, XP055187060
- KRENACS T. ET AL.: 'Application of immunogold-silver staining and immunoenzymatic methods in multiple labelling of human pancreatic Langerhans islet cells' ACTA HISTOCHEMICA vol. 85, 1989, pages 79 - 85, XP008176220

## Description

### Field of invention

The present application relates to the field of immunohistochemistry as well as computer-based analysis of images. In particular, the application provides a method of extracting information from a histological tissue sample and using this information in computer-based analysis of images. More specifically, the present application provides a method of visualizing a plurality of cell types in a microscopic multicell tissue sample.

### Background of the invention

Analysis of a histological tissue sample is commonly used for diagnosis purposes, e.g. analysis of a breast tissue sample for diagnosing breast cancer, or for research purposes, e.g. to study inflammatory cell types in inflammatory conditions such as asthma, atherosclerosis, or inflammatory bowel diseases.

Immunohistochemistry (IHC), whereby a marker molecule (i.e. an antigen, typically a protein) is detected by an antigen-specific antibody, is commonly used to identify cells in histological sections. For some cell types, identification can be obtained with detection of a single cell-specific antigen. However, a significant problem in the field of immunohistochemical identification of tissue cells is the fact that many cells cannot be identified by one marker molecule only. A candidate identification marker for one cell type may be present also on some other cell types and preclude a proper identification. Hence, finding means to identify and exclude confounding cell types is critical for reliable identification of cell types for which no cell-specific marker is available. Ideally, after proper identification it would be desirable to explore the activation status of the identified cells with additional IHC staining that visualizes activation markers, or by in situ hybridization techniques to visualize gene expression.

Any diseased tissue is typically associated with an altered composition of cell populations. For example, in inflamed airways in asthma there is an altered composition of the structural cells that build up the airways, such as epithelial cells, gland cells, blood vessel cells, nerves, etc. In addition, several types of immune cells (i.e. leukocytes) infiltrate the inflamed airways. Indeed,in many diseases, the pathological (i.e. destructive) alterations in the tissue are not caused by just one cell type but rather a complex interaction between several cell types. Hence, when exploring a diseased tissue sample it would often be desirable to visualize several cell populations and tissue structures simultaneously, or to visualize cell populations together with non-cellular structures like invading viruses or extracellular matrix components.

Information about the cellular content in a tissue can be obtained by staining one cell type at the time in serially cut sections. Although this approach provides a good estimation of the content of several cell types in a tissue sample, it does not provide detailed information about the spatial relationship (i.e. physical relationship) between the analysed cell types. This is a major drawback since much of the cross talk between cells requires close, and sometimes direct, physical contact. Thus, it would be desirable to visualize several cell populations within the same section.

IHC techniques are among others, disclosed by J. Lechago et al:" Simultaneous visualtization of two antigens in the same tissue section by combining immunoperoxidase with immunoflourescence techniques.", JOURNAL OF HISTOCHEMISTRY AND CYTOCHEMISTRY, vol.27, no. 9, September 1979, pgs 1221-1225; by Piris J et al: "Immunoperoxidase technique for the identification of gastrin producing cells", JOURNAL OF CLINICAL PATHOLOGY, BJM PUBLISHING GROUP, GB, vol. 27, no 10, October 1974, pgs 798-799; and by Helga Semb's Fund et al: "Supported Comparison of Paired Immunofluorescence and Paired Immunoenzyme Staining Methods Based on Primary Antisera from the Same Species", January 1982, pgs 518-524.

With currently available IHC techniques it is possible to stain up to 3 cell types in one section using multiple-chromogen or multiple immunofluorescence techniques. In common practice, however, often only 2 cell types can be simultaneously detected due to lack of appropriate combinations of primary detection antibodies.

In order to increase the number of markers that can be visualized in one tissue section, new methodological approaches have been developed, such as the SIMPLE technique disclosed in WO 2010/115089 and the MELC technique (Schubert et al., Nature Biotechnology v. 24, pp. 1270-1278). Although powerful, these new types of techniques have primarily been developed for co-localization studies and either involve tissue-destructive procedures, procedures involving destruction of detection groups, or dependence of detection molecule-labeled primary antibodies. The latter limitation, which excludes conventional amplification of the staining sensitivity by using secondary antibodies, renders these approaches too insensitive for detection of many common cell markers in conventional histological sections. Furthermore, since the above mentioned techniques were primarily developed for co-localization studies, they, in similarity to other conventional immunohistochemical protocols, do not handle the problem that many identification markers occasionally also might be present on non-intended cell types.

One approach to properly detect a cell type for which there is no single specific marker is to exclude the confounding cell type by prior IHC detection and encapsulation in detection chromogen. In Andersson et al.: "Novel site-specific mast cell subpopulations in the human lung", Thorax 2009; vol. 64, pp. 297 - 305, the On Line Supplementary data discloses an approach whereby mucosal mast cells (MC_{T}, positive for tryptase) and connective tissue mast cells (MC_{TC}, positive for chymase and tryptase) were separated by a two-stage procedure where the first IHC step detects chymase by the brown chromogen DAB while the second step detect the remaining chymase-negative MCt cells by the red chromogen new fuchsin. While useful for identifying MCt and MCTc populations in a tissue section, a major drawback is that neither of the chromogen-encapsulated cells can be further investigated due to their opaque staining and steric hindrance caused by the detection chromogen. Accordingly, there is a need for an improved and flexible visualization method which enables visualization of a plurality of cell markers in a sample. In particular, there is a need for a non-tissue destructive visualization method which renders it possible to detect further cell markers of interesting cell types of a particular sample in subsequent steps after an initial visualization.

### Summary of the invention

The above mentioned problems are solved in accordance with the present invention by providing a method of visualizing a plurality of cell types in a microscopic multicell tissue sample comprising cells of at least one particular cell type as well as cells of at least one confounding cell type, wherein cells of said at least one particular cell type and cells of said at least one confounding cell type both comprise a second cell marker and cells of the at least one confounding cell type also comprise a first cell marker not present in cells of the at least one particular cell type, comprising the steps of
a) providing a microscopic multicell tissue sample known to comprise cells of at least one confounding cell type as well as cells of at least one particular cell type;
b) incubating the microscopic multicell tissue sample of step a) with a first cocktail of at least one antibody-like ligand, which antibody-like ligand(s) of the cocktail (is)are capable of binding specifically to said first cell marker(s) on confounding cells but not to said second cell markers of cells of said at least one particular cell type, and where the sample is incubated with the first cocktail under conditions favourable for formation of specific bonds between said first cell markers on confounding cells and said ligand(s), resulting in formation of complexes between said first cell markers and said ligand(s);
c) incubating the complexes formed in step b) with a first detecting antibody-like ligand preparation, the first detecting antibody-like ligand being conjugated to a first detection enzyme, said first detecting antibody-like ligand being capable of binding specifically to the antibody-like ligand(s) of said first cocktail, where the incubation is carried out under conditions favourable for formation of specific bonds between said first detecting antibody-like ligand and the antibody-like ligand(s) of said first cocktail, resulting in formation of complexes between said first detecting antibody-like ligand and the complexes formed in step b);
d) adding a first substrate preparation comprising a substrate for said first detection enzyme to the complex formed in step c) and incubating the resulting mixture under conditions that are favourable for formation of a detectable blocking polymer thereby obtaining detectable accumulated blocking polymers within or around confounding cells of said microscopic multicell tissue sample, which blocking polymer prevents access to the second cell marker of the confounding cells;
e) adding a first primary antibody-like ligand preparation, wherein the ligand is capable of binding specifically to the second cell marker on said at least one particular cell type, and incubating the resulting mixture under conditions favourable for formation of complexes between said second cell marker and said first primary antibody-like ligand;
f) adding a first secondary antibody-like ligand preparation to the resulting mixture of step e), the first secondary antibody-like ligand being conjugated to a fluorochrome, said first secondary antibody-like ligand being capable of binding specifically to said first primary antibody-like ligand, and incubating the resulting mixture under conditions favourable for formation of specific bonds between said first primary and first secondary antibody-like ligands;
g) removing unbound secondary antibody-like ligands, for instance through a washing step;
h) chemically fixating complexes of bound primary and secondary antibody-like ligands;
i) exposing cells of the resulting sample from step h) to a group of at least one fluorochrome-labelled antibody-like ligand preparation(s), each preparation of said group of at least one fluorochrome-labelled antibody-like ligand preparation(s) being capable of specifically binding to a unique further cell marker associated with at least one particular cell type of interest, each preparation of said group of at least one fluorochrome-labelled antibody-like ligand preparation(s) having a unique fluorochrome capable of sending out a unique fluorescence signal;
j) removing unbound fluorochrome-labelled ligands, for example through a washing step.
   and
k) during the course of the method, visualizing said at least one particular cell type and said at least one confounding cell types by detecting fluorescence signals obtained from one or more fluorochrome(s), and by detecting the blocking polymer that has been formed within or around said at least one confounding cell type.

### Detailed description of the invention

The present invention provides a visualization method which, given the current needs and technical limitations, provides significantly improved information of a cell population in tissues that may be diseased. Accordingly, the invention provides a novel methodological approach whereby the cell population of interest is: 1) properly identified; 2) subsequently stained for e.g. activation markers; and 3) visualized together with other cell populations and non-cellular structures in the same tissue section. Ideally, apart from these criteria, any such technique should be capable of analyzing an entire large section of samples and providing detailed information about all marked individual cells such as their spatial coordinates in the tissue, their size and shape parameters etc.

Accordingly, in a first aspect and in its broadest sense, the present invention provides a method of visualizing a plurality of cell types in a microscopic multicell tissue sample comprising cells of at least one particular cell type as well as cells of at least one confounding cell type, wherein cells of said at least one particular cell type and cells of said at least one confounding cell type both comprise a second cell marker and cells of the at least one confounding cell type also comprise a first cell marker not present in cells of the at least one particular cell type, comprising the steps of
a) providing a microscopic multicell tissue sample known to comprise cells of at least one confounding cell type as well as cells of at least one particular cell type;
b) incubating the microscopic multicell tissue sample of step a) with a first cocktail of at least one antibody-like ligand, which antibody-like ligand(s) of the cocktail (is)are capable of binding specifically to said first cell marker(s) on confounding cells but not to said second cell markers of cells of said at least one particular cell type, and where the sample is incubated with the first cocktail under conditions favourable for formation of specific bonds between said first cell markers on confounding cells and said ligand(s), resulting in formation of complexes between said cell markers and said ligand(s);
c) incubating the complexes formed in step b) with a first detecting antibody-like ligand preparation, the first detecting antibody-like ligand being conjugated to a first detection enzyme, said first detecting antibody-like ligand being capable of binding specifically to the antibody-like ligand(s) of said first cocktail, where the incubation is carried out under conditions favourable for formation of specific bonds between said first detecting antibody-like ligand and the antibody-like ligand(s) of said first cocktail, resulting in formation of complexes between said first detecting antibody-like ligand and the complexes formed in step b);
d) adding a first substrate preparation comprising a substrate for said first detection enzyme to the complex formed in step c) and incubating the resulting mixture under conditions that are favourable for formation of a detectable blocking polymer thereby obtaining detectable accumulated blocking polymers within or around confounding cells of said microscopic multicell tissue sample, which blocking polymer prevents access to the second cell marker of the confounding cells;
e) adding a first primary antibody-like ligand preparation, wherein the ligand is capable of binding specifically to the second cell marker on said at least one particular cell type, and incubating the resulting mixture under conditions favourable for formation of complexes between said second cell marker and said first primary antibody-like ligand;
f) adding a first secondary antibody-like ligand preparation to the resulting mixture of step e), the first secondary antibody-like ligand being conjugated to a fluorochrome, said first secondary antibody-like ligand being capable of binding specifically to said first primary antibody-like ligand, and incubating the resulting mixture under conditions favourable for formation of specific bonds between said first primary and first secondary antibody-like ligands;
g) removing unbound secondary antibody-like ligands, for instance through a washing step;
h) chemically fixating complexes of bound primary and secondary antibody-like ligands;
i) exposing cells of the resulting sample from step h) to a group of at least one fluorochrome-labelled antibody-like ligand preparation(s), each preparation of said group of at least one fluorochrome-labelled antibody-like ligand preparation(s) being capable of specifically binding to a unique further cell marker associated with at least one particular cell type of interest, each preparation of said group of at least one fluorochrome-labelled antibody-like ligand preparation(s) having a unique fluorochrome capable of sending out a unique fluorescence signal;
j) removing unbound fluorochrome-labelled ligands, for example through a washing step.
   and
k) during the course of the method, visualizing said at least one particular cell type and said at least one confounding cell types by detecting fluorescence signals obtained from one or more fluorochrome(s), and by detecting the blocking polymer that has been formed within or around said at least one confounding cell type.

As disclosed herein, the term "molecular detection means" relates to a bi-functional aggregate or conjugate comprising a first part capable of specific binding to a particular cell marker, and a second part capable of generating a detectable response. Many different types of molecular detection means could be used in the present invention, for example such molecular detection means that are suitable for use in immunohistochemistry, is situ hybridization and enzyme histochemistry methods.

Typically, said first part capable of specific binding to a particular cell marker could be an antibody or a fragment thereof, or a nucleic acid molecule such as a DNA molecule or an RNA molecule or a nucleic acid derivative such as PNA. Typically, said second part capable of generating a detectable response could be an enzyme capable of catalyzing formation of a blocking polymer, or a chemical compound capable of generating some kind of detectable fluorescence signal, typically a fluorochrome, when induced by suitable excitating light.

A molecular detection means could, in its simplest embodiment, be comprised of a first part such as an antibody or a nucleic acid molecule to which the second part such as an enzyme or a fluorochrome has been bound. Alternatively, a suitable molecular detection means could be a complex comprising a first entity, typically a monoclonal or a polyclonal antibody specifically binding to a particular cell marker, a second entity, typically a monoclonal or a polyclonal antibody, specifically binding to the first entity, and a third entity bound to the second entity, where said third entity is an enzyme capable of catalyzing formation of a blocking polymer, or a chemical compound capable of generating some kind of detectable fluorescence signal, typically a fluorochrome, when induced by suitable excitating light.

As disclosed herein, the "chemically fixating" relates to methods for covalently binding the molecular detection means to the sample to which the molecular detection means have specifically bound in order to prevent that the molecular detection means is washed away. Typically cross-linking molecules react with the sample as well as the molecular detection means. The skilled person is familiar with suitable cross-linking molecules and methods.

As disclosed herein, the term "visualize" relates to producing at least one image of the sample where blocking and other chromogen polymers as well as fluorescence signals are clearly visible. Typically, the visualization is carried out in two steps; firstly generation of bright field microscopic image/s of the stained chromogen/s. Preferably, the image is a digital photo. Secondly, each detection fluorochrome is visualized and by immunofluorescence microscopy where images are captured using wavelength-specific filters. Also here the obtained images are preferably, but not limited to, digital images. For each marker the analysed area may be enhanced by combination of images from neighbouring or overlapping tissue areas by digital stitching of images. The skilled person is aware of such techniques. Images used for producing a composite image may in some embodiments be obtained at any time when carrying out the visualization method of the invention. Using computerized image analysis, information from the digital photos is used to extract raw data for each marker. Typically, positive marker areas in the sections are detected and segmented out by its specific colour and intensity values. Once the positive cells have been segmented information of the x,y coordinates, shape, and intensity can easily be extracted by known methods within the field of computerized image analysis. The segmented cells are also used to generate a novel type of interactive composite images where the information about the analysed cells is presented in an unprecedented detail through combining data about all analysed markers. Typically, the marker data are pasted into a template image displaying the background tissue structure. A person skilled in the field will understand that the generated raw data about marker areas can be used to calculate the percentage of the cell population of interest that express the analyzed activation markers as well as the spatial relationship of the visualized cells.

As disclosed herein, the term "microscopic multicell tissue sample" relates to any histological tissue sample that comprises a multitude of cells, that could be examined by microscope-based visualisation techniques, and where the cells of the sample could be examined by exposing cell markers of the cells to specific ligands.

As disclosed herein, the terms "cell type" and "particular cell type" in their broadest sense relate to any type of cell in a microscopic multicell tissue sample. However, a "particular cell type" is typically a cell type that could be present in a tissue sample but does not constitute a substantial part of it.

As disclosed herein, the term "confounding cells" relates to cells partially characterized by the same cell marker(s) as a particular cell type of interest and that may occur in larger or smaller numbers in the tissue sample compared with the cell type(s) of interest. In accordance with the present invention, the confounding cells of a particular sample are excluded by detecting them with polymer formation before detecting the particular cell type(s) using fluorescence detection.

As disclosed herein the term "preparation" relates to a composition, typically a buffered aqueous solution, containing a particular ingredient.

As disclosed herein, the term "antibody-like ligands" relates to antibodies, antibody fragments or antibody mimetics. Suitable antibodies are preferably monoclonal antibodies, but may also include "mono-specific" polyclonal antiserum. Suitable antibody fragments include, without limitation, Fab fragments, F(ab)₂ fragments, Fab' fragments, F(ab')₂ fragments, Fd fragments, Fd' fragments, Fv fragments, 61-residue subdomains of the antibody heavy-chain variable domain known as minibodies (Pessi et al., Nature 362:367-369 (1993), and domain antibodies (dAbs)(Holt et la., Trends Biotechnol. 21:484-90 (2003)). Many of these antibody fragments can be made by conventional procedures, such as proteolytic fragmentation procedures, as described in J. Goding, Monoclonal Antibodies: Principles and Practice 98-118 (1984). Suitable antibody mimetics include, without limitation, those known as monobodies, which are derived from the tenth human fibronectin type III domain (Koide et al., J. Mol. Biol. 284:1141-1151 (1998); Koide et al., Proc. Natl. Acad. Sci. USA 99: 1253-1258 (2002)); and those known as affibodies, which are derived from the stable α-helical bacterial receptor domain Z of staphylococcal protein A (Nord et al., Nat. Biotechnol. 15(8):772-777 (1997)). Variations in these antibody mimetics can be created by substituting one or more domains of these polypeptides and then screening the modified monobodies or affibodies for specificity for binding to marker proteins of interest.

As disclosed herein, the term "cell marker" relates to a specific structure that, depending on cell type, may occur more or less specifically, often on the surface of a cell of the cell type, but sometimes also within the cell. Typically, a cell marker is a receptor capable of binding specifically to a particular target molecule. The cell marker may be a protein, a glycoprotein, or a carbohydrate, a nucleic acid, a lipid or another type of naturally occurring biological molecule. The skilled person knows abbout such cell markers and cell types on which they occur. Presence of one or more such cell markers may indicate that the cell belongs to a certain class or type of cells.

As disclosed herein, the term "non-cellular structure" relates to structures occurring in the extracellular space of the tissue section. The non-cellular structure could be components of the extracellular matrix (e.g. collagen fibres, bone and hair), plasma proteins, amylin, or exogenous matters like invading microorganisms (e.g. bacteria, viruses, parasites and fungi).

As disclosed herein, the terms "cocktail of antibody-like ligands" or just "cocktail" relate to a preparation comprising at least one antibody-like ligand. The antibody-like ligand(s) of the cocktail are capable of specific binding to specific cell markers on confounding cells deemed to be present in the microscopic multicell tissue sample. Moreover, all antibody-like ligands of the cocktail comprise a common epitope rendering it possible to provide a single monoclonal antibody capable of binding specifically to all ligands of the cocktail.

As disclosed herein, the term "detecting antibody-like ligand" relates to an antibody-like ligand covalently bound to a detecting enzyme. As disclosed herein, the term "detection enzyme" relates to an enzyme capable of catalyzing formation of an opaque and/or coloured polymer in presence of certain substrates under suitable conditions. Examples of suitable detection enzymes are alkaline phosphatase and peroxidases, such as horseradish peroxidase. Examples of suitable substrates for such enzymes are 3,3'-diaminobenzidine (DAB), betazoid DAB, Deep Space Black, Bajoran Purple, Warp Red, Feringi Blue, Vulcan Fast Red and Vina green.

Rather than being directly bound to the secondary antibody, the detection enzyme may also, in an alternative embodiment, be attached to molecules commonly used to amplify the detection signal. Examples of such amplification systems is the use of biotin-labelled secondary antibodies and subsequent incubation with biotin-binding streptavidin that is labelled with detection enzyme. Other common detection systems are the polymer-based amplification techniques ENVision™ system by Dako and the Polink-2 System by GBI labs.

As disclosed herein, the term "primary antibody-like ligand" relates to an antibody-like ligand capable of specifically binding to a cell marker associated with a particular cell type of interest. The term "secondary antibody-like ligand" relates to an antibody-like ligand capable of binding specifically to an epitope of the primary antibody-like ligand. In case more than one pair of primary and secondary antibody-like ligands are used, the secondary antibody-like ligand of a particular pair is only able to bind to the primary antibody-like ligand of the same pair. As disclosed herein, the term "detection group" relates to a group capable of generating a specific signal when induced by a specific action. Typically, the detection group is a fluorochrome, capable of emitting light of a specific wave length after exposure to UV light. In case more than one pair of primary and secondary antibody-like ligands are used, each particular secondary antibody-like ligand has a specific detection group capable of emitting a specific signal.

In one embodiment of the first aspect of the invention, a second cocktail of at least one antibody-like ligand(s) is used together with the first cocktail in step b) and a second detecting antibody-like ligand preparation is used in step c). The antibody-like ligand(s) of the second cocktail are capable of specifically binding to other cell marker(s) of the confounding cell types than the ligands of the first cocktail. The second detecting antibody-like ligand is capable of binding specifically to the antibody-like ligands of the second cocktail. The second detecting antibody-like ligand has a second detection enzyme, and a second substrate preparation comprising a substrate for said second detection enzyme is added in step d) resulting in accumulation of an additional blocking polymer in the vicinity of confounding cells binding to the second cocktail. Accumulation of additional blocking polymer is detected in step h).

In another embodiment of the first aspect of the invention, the following steps are carried out after step c) and before step d):
1) incubating the resulting mixture of step c) with a second cocktail of antibody-like ligands, wherein the antibody-like ligand(s) of the second cocktail is/are capable of specifically binding to other cell marker(s) of other confounding cell types than said first cocktail, under conditions allowing ligands of the second cocktail to bind to said other cell markers; and
2) incubating the resulting mixture of step 1) with a second detecting antibody-like ligand the second detecting antibody-like ligand being capable of binding specifically to the antibody-like ligand(s) of the second cocktail, said second detecting antibody-like ligand having a second detection enzyme;
and wherein a second substrate preparation comprising a substrate for said second detection enzyme is added in step d) resulting in accumulation of an additional blocking polymer within or around of confounding cells binding to the second cocktail, and where said accumulation of additional blocking polymer is detected in step h).

Preferably, steps 1) and 2) are repeated with a third cocktail of antibody-like ligands, a third detecting antibody-like ligand, and a third substrate preparation comprising a substrate for said third detection enzyme, said the antibody-like ligand(s) of the third cocktail are capable of specifically binding to other first cell marker(s) of other confounding cell type than said first and second cocktails, said third detecting antibody-like ligand being capable of binding specifically to the antibody-like ligand(s) of the third cocktail, said third detecting antibody-like ligand having a third detection enzyme, said third substrate preparation comprising a substrate for said third detection enzyme, and where said accumulation of additional blocking polymer is detected in step h).

Preferably, steps 1) and 2) are repeated with a fourth cocktail of antibody-like ligands, a fourth detecting antibody-like ligand, and a fourth substrate preparation comprising a substrate for said fourth detection enzyme, said the antibody-like ligand(s) of the fourth cocktail are capable of specifically binding to other first cell marker(s) of other confounding cell type than said first, second, and third cocktails, said fourth detecting antibody-like ligand being capable of binding specifically to the antibody-like ligand(s) of the fourth cocktail, said fourth detecting antibody-like ligand having a fourth detection enzyme, said fourth substrate preparation comprising a substrate for said fourth detection enzyme, and where said accumulation of additional blocking polymer is detected in step h).

Preferably, the colour of the accumulated blocking polymer formed of the first substrate preparation is dark and/or opaque and that the colours of subsequently formed accumulated blocking polymers are gradually getting brighter and/or less opaque.

In a preferred embodiment, a plurality of pairs of antibody-like ligand preparations are used in steps e) - h), each of said pairs being used for detecting one particular cell type of interest out of the plurality of particular cell types, each pair comprising
a certain primary antibody-like ligand preparation comprising a ligand capable of specifically binding to a unique cell marker associated with a particular cell type of interest; and
a certain secondary antibody-like ligand preparation comprising a ligand capable of specifically binding to said certain primary antibody-like ligand, which secondary antibody-like ligand is covalently bound to a fluorochrome capable of sending out a unique fluorescence signal.

In a preferred embodiment of the first aspect of the invention, the detection enzyme is chosen from the group of alkaline phosphatase and a peroxidase, such as horseradish peroxidase.

Preferably, the substrate(s) added in step d) is chosen from the group of 3,3'-diaminobenzidine (DAB), Betazoid DAB, Deep Space Black, Bajoran Purple, Warp Red, Ferangi Blue, Vulcan Fast Red, and Vina green.

Preferably, the detection group(s) is(are) (a) fluorochrome(s), such as the fluorochromes of the group of Alexa Fluor fluorochromes, e.g. Alexa Fluor 680, Alexa Fluor 488, Alexa Fluor 350 and Alexa Fluor 555; the Cy fluorochromes such as Cy2, and Cy7; the DyLight fluorochromes e.g. DyLight 350, DyLight 488, DyLight 555, DyLight750; TRITC, FITC, Texas Red.

In one embodiment of the first aspect of the invention, the visualization step h) is carried out by obtaining one or more photographic images of the sample.

Preferably, the obtained images are digitalized according to known principles and combined into a composite image.

Preferably, at least 4 different cell markers or non-cellular structures including markers representing confounding cells and particular cell types are detected, and more preferably at least 5 different cell markers are detected.

### Brief description of the drawings

The present invention will now be described with reference to the enclosed figures, in which:
Figures 1a - 1c schematically discloses the principle of blocking out a particular cell marker on confounding cells followed by detecting a cell type of interest using the particular cell marker as specific marker of the cell type; and
Figure 2 discloses a more detailed example of the method according to the invention where six different cell types are detected in a tissue sample and an interactive composite image is prepared.

### Basic principles of the invention

Proteins are commonly and readily detected in histological tissue sections by immunohistochemistry, a technique that uses antibodies that bind specifically to the protein of interest. Bound antibodies can be visualized by enzymes that produce a coloured reaction product (bright field IHC) or by molecules producing fluorescence light when exposed to UV light (immunofluorescence IHC).

Different cell types can be detected by IHC detection of cell-specific proteins (cell markers). As already mentioned, a significant problem in the field of immunohistochemical identification of tissue cells is the fact that many cells cannot be identified by one cell marker only. For example, a candidate identification marker for one cell type may also be present on some other cell types and preclude a proper idenfification. Such "other cell types" are referred to herein as "confounding cells".

Furthermore, although it would be desirable to detect many markers and proteins (i.e. different cells or markers) within the same tissue section, traditional IHC techniques only allow detection of 2 (maximum 3) different proteins in the same section.

Through an innovative combination and modifications of known bright field and fluorescence IHC staining protocols, a new method is described whereby improved cell identification is achieved by physical exclusion of any confounding cell characterization by multiple IHC staining. Apart from providing enhanced cell identification, the present method is also capable of simultaneous detection of 8 cell populations in one automated staining run. Another advantage with a physical exclusion of confounding cells prior to identification and further examination of the cell population of interest is that the primary antibodies used to detect confounding cells and cells ofinterest can be of the same isotype. The method can be highly automated and is suitable for large scale and time-efficient generation of high quality multiple staining patterns.

The method provides a way of taking advantage of the fact that some cell markers may be found on more than one cell type, and that there are cell types that cannot be characterized by any specific cell markers. Table 1 below lists some examples of immune cell markers which are suitable for use in the present invention, and cells expressing them:

**Table 1: Cell markers for IHC detection of cells in histological sections**

| **Cell marker** | **Primary cell** |
|---|---|
| CD20 | B lymphocytes |
| CD3 | T lymphocytes |
| ECP (EG2) | Eosinophils |
| CD11c | Myloid dendritic cells, Macrophages, neutrophils |
| Tryptase | Mast cells |
| CD68 | Macrophages/monocytes (and occational neutrophils), basophils, large lymphocytes |
| MPO | Neutrophils |
| CD163 | Most tissue macrophages (but not in follicular macrophages) and Langerhan cells |
| CD123 | Plasmacytoid dendritic cells, macrophages/monocytes, neutrophils, eosinophils |
| CD45 | All leucocytes |

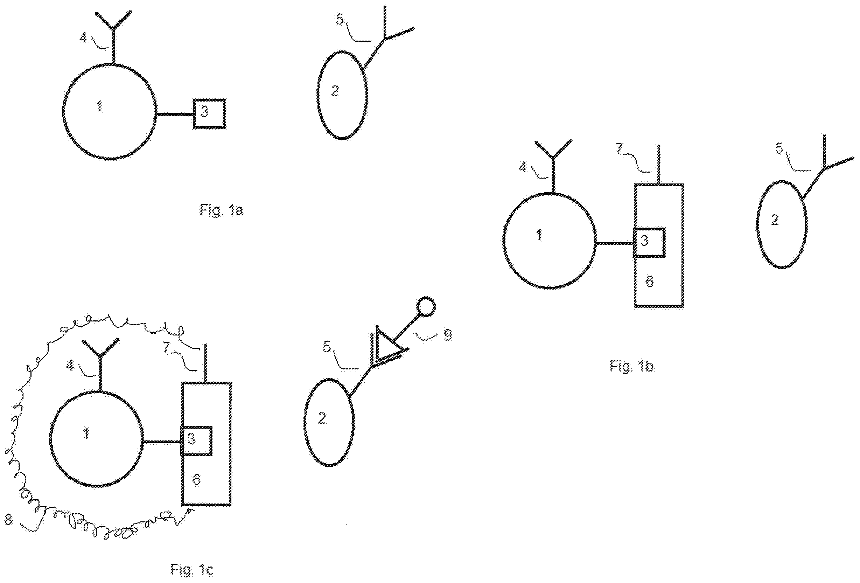

This principle is shown in Figures 1a - 1c. Figure 1a discloses a tissue sample comprising one cell 1 of a confounding cell type and one cell 2 of a cell type of interest. The confounding cell 1 has a first cell marker 4 and a second cell marker 3. The cell 2 of the cell type of interest also has the first cell marker 5, but it lacks the second cell marker. In Figure 1b, a first molecular detection means 6 has been bound to the first cell marker 3. It should be noted that the first molecular detection means comprises an enzyme 7. Figure 1c shows the final detection step of the method. The enzyme 7 of the first molecular detection means 6 has catalyzed formation of a blocking polymer 8 preventing access to the second cell marker 4 of the confounding cell 1. On the other hand, the second cell marker 5 on the cell type of interest has not been blocked by the polymer 8. Hence, a second molecular detection means 9 comprising a fluorochrome emitting a detectable fluorescence signal after excitation with suitable light may bind to the the second cell marker 5 of the cell type of interest. Consequently, the second cell marker can be used as a basis of specific detection of the cell type of interest despite the fact that the second cell marker is present in both cells of the cell type of interest and in confounding cells of the sample. The second molecular detection means is comprised of a first entity (typically an antibody) specifically binding to the the second cell marker, and a second entity comprising a fluorochrome. The second entity may be covalently bound to the first entity. In some embodiments, the second entity may typically be an antibody specifically binding to the first entity, to which a fluorochrome is covalently bound. In some embodiments, the second molecular detection means is chemically fixated to the sample in order to cross-link the previously bound secondary antibodies in order to prevent their dissociation and diffusion to primary antibodies added in possible subsequent steps.

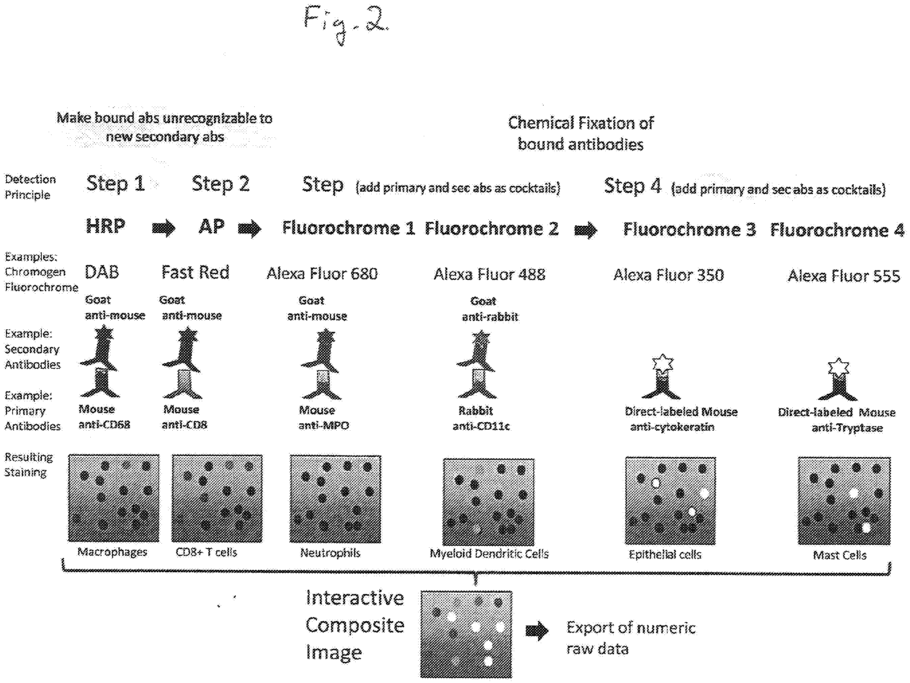

A more complex embodiment of the present invention is shown in Figure 2. Through an innovative combination and modifications of known bright field and fluoroscence IHC staining protocols, Fig. 2 discloses an embodiment of the present invention which, apart from yielding improved cell identification by efficiently excluding any confounding cells, has the capacity to simultaneously stain up to eight antigens (e.g. cell populations) in one automated IHC protocol. Note that any of the immunohistochemical steps in Figure 2 can be replaced with other means for marker molecule detection. For example, in situ hybridization with chromogenic detection may be used in any of steps 1A and 1B. Similarly, in situ hybridization with fluorescence detection may be used in any of the steps 2 or 34 in Figure 2. The described embodiment can be highly automated and is suitable for large scale and time-efficient generation of high quality multiple staining patterns. Any such kind of detailed information of for instance leucocyte infiltration pattern is likely to have multiple important applications ranging from basic research, drug validation, disease phenotyping and clinical diagnosis.

A microscopic multicell tissue sample suspected of containing macrophages, CD8+ T cells, neutrophils, myeloid dendritic cells, epithelial cells and mast cells is to be analyzed. The macrophages are regarded as confounding cells because the cell type comprise several cell markers that are candidate markers for e.g. myloid dendritic cells (e.g. CD11c). In a first step, the sample is exposed to mouse anti-CD68 antibodies followed by goat-anti-mouse antibodies marked with the enzyme horseradish peroxidase (HRP). Presence of macrophages is indicated by formation of opaque polymer when the enzyme catalyses a reaction involving 3,3'-diaminobenzidine (DAB). In case further primary antibodies from same origin are to be used for detecting another cell marker, a stripping buffer having an appropriate combination of pH, osmolarity, temperature, detergents and/or denaturating agents (Harlow & Lane (1999) Using Antibodies: A Laboratory Manual. Cold Spring Harbor, NY, Cold Spring Harbor Laboratory Press.) could be added. A non-restricted example of such an eluation step is incubation of the slides for 15 min in 25 mM glycine-HCI, pH 2 in 10 % SDS. The sample is also exposed to mouse anti-CD8 antibodies followed by exposure to goat anti-mouse antibodies conjugated to the enzyme alcaline phosphatase (AP). Presence of CD8+ T cells is shown by adding Fast Red. Now that macrophages are blocked, it is possible to display neutrophils by adding mouse anti-MPO antibodies followed by addition of goat anti-mouse antibodies marked with fluorochrome Alexa Fluor 680. Similarly, presence of myeloid dendritic cells can be shown by adding rabbit anti CD11c antibodies followed by goat anti-rabbit antibodies marked with fluorochrome Alexa Fluor 488. A chemical fixation of bound antibodies may now be carried out, for instance by exposure to 3 % buffered formaldehyde solution for 15 minutes. The purpose of the chemical fixation is to cross-link the previously bound secondary antibodies in order to prevent their dissociation and diffusion to primary antibodies added in subsequent steps. After chemical fixation of bound antibodies it is possible to detect presence of epithelial cells by adding mouse anti-cytokeratin antibodies directly labelled with fluorochrome Alexa Fluor 350, and to detect presence of mast cells by adding mouse anti-tryptase antibodies directly labelled with fluorochrome Alexa Fluor 555. An image is obtained after each staining, and finally an interactive composite image is obtained.

The invention will now be further described with reference to the following example of identification protocols:

### Example 1: Protocol for identification and characterization of tissue Neocytes (also referred to as Type 2 innate lymphoid cells)

Background: A new type of IL-13 producing and ST2 receptor-expressing innate effector leukocyte, the neocyte, was recently discovered (Neill et al., Nature 2010 Apr 29; 464(7293):1367-70) and has been proposed as major regulator of allergic and infectious diseases. As a result, there is a lot of medical and commercial interest in further explorations of neocytes as pharmacological targets for novel inti-inflammatory drugs. Currently, the research on neocytes is hampered by lack of methods for their proper identification in human tissues. This problem is, however, solved by using the following protocol combining appropriate exclusion and positive detection markers.

| | **Primary exclusion** | **Separate parallel staining** | IF step 1 | IF step 2 | IF step 3 | Nuclear counter staining |
|---|---|---|---|---|---|---|
| Primary antibodies | Exclusion cocktail: CD3, CD20, CD138, CD68, Bosogranulin ECP, MPO, CD11c, Tryptase, BDCA2, (+ selected progenitor markers) | Podoplanin (DP-40) | Mouse anti-IL-13 | Rabbit anti-ST2 | Alexa 645-conjug. Mouse anti-CD45 | DNA-binding fluorochrome Hoechst 3222 |
| Secondary antibody/detection system | C-IHC with HRP and chromogenic visualization (e.g. brown precipitation with DAB) | C-IHC with AP and chromogenic detection (e.g. Fast Red) | Goat anti-mouse (Alexa448) | Goat anti-rabbit (Alexa 555) | NA | NA |
| Resulting staining | Opaque brown preci-pitation in non-nuocyte leukocyte populations | Parallel staining of lymphatic vessels | Identification of IL-13 producing cells | Identification of ST2-expressing cells | Visualization of "non-traditional" leukocytes | Background visualization of cell nuclei |

The present protocol was used to identify non-brown cells that did not belong to any traditional leukocyte population (i.e. lack of exclusion markers for common T cells, B cells, macrophages, neutrophils etc.) but yet are leukocytes (defined by CD-45 positivity). IL-13 and ST" are molecules that are known to be present on neucytes (also on some other cells but these have been eliminated in the first step). Hence, neucytes were identified in the tissue as non-traditional CD45+ leukocytes that also co-stain for IL-13 and ST2L. The expression patterns of markers of neocytes is under intense investigation. It is therefore of note that detection of neocytes may also be carried out using the same methodological principle but with other sets of newly discovered "markers" that are also present on confounding cells. Likewise, the immunohistochemical detection of IL-13 protein in this example may be replaced with e.g. visualization of IL-13 mRNA production by in situ hybridization.

### Example 2: Protocol for simultaneous identification of BDCA3+ and BDCA3-myloid dendritic cells, their proliferation status and spatial relationship to invading influenza A viruses.

Background: The capture and presentation of foregin matters to the immune system is carried out by antigen presenting dendritic cells. Dendritic cells exist in many sub-types, many of which are difficult to detect in tissue sections due to lack of markers. For example, attempts have been made to detect myloiddendritic cells by their expression of the surface marker CD11c. This molecule is however expressed on other cell types in the tissue, e.g. on monocytes, macrophages, neutrophils, NK cells, and some B lymphocytes. After excluding these confounding cells in the primary exclusion step of the protocol below , CD11c can safely be used to detect myloid dendritic cells. The protocol has furthermore the capacity to identify the two major subsets of myloid dendritic cells, namely BDCA3-positive myloid DCs and BDCA3-negative DCs (as well as to visualize their detailed spatial relationship to sources of antigen, e.g. invading viruses).

| | **Primary exclusion** | **Separate parallel staining** | IF step 1 | IF step 2 | IF step 3 | Nuclear counter staining |
|---|---|---|---|---|---|---|
| Primary antibodies | Exclusion cocktail: CD68, CD163, CD57, MPO, CD20 | Goat antiinfluenza A | Mouse anti-BDCA3 | Rabbit anti-Ki67 | Alexa-conjug. mouse anti-CD11c | DNA-binding fluorochrome Hoechst 3222 |
| Secondary antibody/ detection system | C-IHC with HRP and chromogenic visualization (e.g. brown precipitation with DAB) | C-IHC with AP and chromogenic detection (e.g. Fast Red) | Goat anti-mouse (Alexa 448) | Goat anti-rabbit (Alexa 555) | NA | NA |
| Resulting staining | Opaque brown precipitation in non-nuocyte leukocyte populations | Parallel staining of Influenza A viruses | Identification of BDCA3+ and DBCA2-myloid DCs | Identification of cell proliferation | Visualization of myloid cells: CD68-, CD163-, CD11c+ cells | Background visualization of cell nuclei |

In the exclusion step, all non-myloid DCs cells expressing CD11c were eliminated from further analysis. Myloid DCs were thereafter safely identified by their CD11c expression. Technically, myloid DCs were thus defined as CD68⁻, CD163⁻, MPO⁻, CD57⁻, CD20⁻, CD11c⁺ cells. These myloid DCs were also categorized with this protocol into the two major myloid DC subsets based on their expression of the surface marker BDCA-3. Technically, the protocol thus provided a simultaneous visualization of CD68⁻, CD163⁻, MPO⁻, CD57⁻, CD20⁻, CD11c⁺ BDCA3⁻ myloid DCs and CD163⁻, MPO⁻, CD57⁻, CD20⁻, CD11c⁺ BDCA3⁺ myloid DCs and their exact spatial relationship to invading influenza A viruses.

## Claims

1. A method of visualizing a plurality of cell types in a microscopic multicell tissue sample comprising cells of at least one particular cell type as well as cells of at least one confounding cell type, wherein cells of said at least one particular cell type and cells of said at least one confounding cell type both comprise a second cell marker and cells of the at least one confounding cell type also comprise a first cell marker not present in cells of the at least one particular cell type, comprising the steps of:
a) providing a microscopic multicell tissue sample known to comprise cells of at least one confounding cell type as well as cells of at least one particular cell type;
b) incubating the microscopic multicell tissue sample of step a) with a first cocktail of at least one antibody-like ligand, which antibody-like ligand(s) of the cocktail (is)are capable of binding specifically to said first cell marker(s) on confounding cells but not to said second cell markers on cells of said at least one particular cell type, and where the sample is incubated with the first cocktail under conditions favourable for formation of specific bonds between said first cell markers on confounding cells and said ligands, resulting in formation of complexes between said first cell markers of said cells and said ligands;
c) incubating the complexes formed in step b) with a first detecting antibody-like ligand preparation, the first detecting antibody-like ligand being conjugated to a first detection enzyme, said first detecting antibody-like ligand being capable of binding specifically to the antibody-like ligands of said first cocktail, where the incubation is carried out under conditions favourable for formation of specific bonds between said first detecting antibody-like ligand and the antibody-like ligand(s) of said first cocktail, resulting in formation of complexes between said first detecting antibody-like ligand and the complexes formed in step b);
d) adding a first substrate preparation comprising a substrate for said first detection enzyme to the complex formed in step c) and incubating the resulting mixture under conditions that are favourable for formation of a detectable blocking polymer thereby obtaining detectable accumulated blocking polymers within or around confounding cells of said microscopic multicell tissue sample, which blocking polymer prevents access to the second cell marker of the confounding cells;
e) adding a first primary antibody-like ligand preparation, wherein the ligand is capable of binding specifically to the second cell marker on said at least one particular cell type, and incubating the resulting mixture under conditions favourable for formation of complexes between said second cell marker and said first primary antibody-like ligand;
f) adding a first secondary antibody-like ligand preparation to the resulting mixture of step e), the first secondary antibody-like ligand being conjugated to a fluorochrome, said first secondary antibody-like ligand being capable of binding specifically to said first primary antibody-like ligand, and incubating the resulting mixture under conditions favourable for formation of specific bonds between said first primary and first secondary antibody-like ligands;
g) removing unbound secondary antibody-like ligands, for instance through a washing step;
h) chemically fixating complexes of bound primary and secondary antibody-like ligands;
i) exposing cells of the resulting sample from step h) to a group of at least one fluorochrome-labelled antibody-like ligand preparation(s), each preparation of said group of at least one fluorochrome-labelled antibody-like ligand preparation(s) being capable of specifically binding to a unique further cell marker associated with at least one particular cell type of interest, each preparation of said group of at least one fluorochrome-labelled antibody-like ligand preparation(s) having a unique fluorochrome capable of sending out a unique fluorescence signal;
j) removing unbound fluorochrome-labelled ligands, for example through a washing step; and
k) during the course of the method, visualizing said at least one particular cell type and said at least one confounding cell type by detecting fluorescence signals obtained from one or more fluorochrome(s), and by detecting the blocking polymer that has been formed within or around said at least one confounding cell type.

2. A visualization method according to claim 1, wherein a second cocktail of at least one antibody-like ligand(s) is used together with the first cocktail in step b), wherein the antibody-like ligand(s) of the second cocktail are capable of specifically binding to other cell marker(s) of the confounding cells types than the ligands of said first cocktail and wherein a second detecting antibody-like ligand preparation is used in step c), the second detecting antibody-like ligand being capable of binding specifically to the antibody-like ligand(s) of the second cocktail, said second detecting antibody-like ligand having a second detection enzyme, and wherein a second substrate preparation comprising a substrate for said second detection enzyme is added in step d) resulting in accumulation of an additional blocking polymer within or around confounding cells binding to the second cocktail, and where said accumulation of additional blocking polymer is detected in step h).

3. A visualization method according to claim 1 wherein the following steps are carried out after step c) and before step d):
1) incubating the resulting mixture of step c) with a second cocktail of antibody-like ligands, wherein the antibody-like ligand(s) of the second cocktail is/are capable of specifically binding to other cell marker(s) of other confounding cell type than said first cocktail, under conditions allowing ligands of the second cocktail to bind to said other cell markers; and
2) incubating the resulting mixture of step 1) with a second detecting antibody-like ligand, the second detecting antibody-like ligand being capable of binding specifically to the antibody-like ligand(s) of the second cocktail, said second detecting antibody-like ligand having a second detection enzyme;
and wherein a second substrate preparation comprising a substrate for said second detection enzyme is added in step d) resulting in accumulation of an additional blocking polymer within or around of confounding cells binding to the second cocktail, and where said accumulation of additional blocking polymer is detected in step h).

4. A visualizing method according to claim 3, wherein steps 1) and 2) are repeated with a third cocktail of antibody-like ligands, a third detecting antibody-like ligand, and a third substrate preparation comprising a substrate for said third detection enzyme, said the antibody-like ligand(s) of the third cocktail are capable of specifically binding to other first cell marker(s) of other confounding cell type than said first and second cocktails, said third detecting antibody-like ligand being capable of binding specifically to the antibody-like ligand(s) of the third cocktail, said third detecting antibody-like ligand having a third detection enzyme, said third substrate preparation comprising a substrate for said third detection enzyme, and where said accumulation of additional blocking polymer is detected in step h).

5. A visualizing method according to claim 4, wherein steps 1) and 2) are repeated with a fourth cocktail of antibody-like ligands, a fourth detecting antibody-like ligand, and a fourth substrate preparation comprising a substrate for said fourth detection enzyme, said the antibody-like ligand(s) of the fourth cocktail are capable of specifically binding to other first cell marker(s) of other confounding cell type than said first, second, and third cocktails, said fourth detecting antibody-like ligand being capable of binding specifically to the antibody-like ligand(s) of the fourth cocktail, said fourth detecting antibody-like ligand having a fourth detection enzyme, said fourth substrate preparation comprising a substrate for said fourth detection enzyme, and where said accumulation of additional blocking polymer is detected in step h).

6. A visualization method according to any of claims 3 - 5, wherein the colour of the accumulated blocking polymer formed of the first substrate preparation is dark and/or opaque and that the colours of subsequently formed accumulated blocking polymers are gradually getting brighter and/or less opaque.

7. A visualization method according to any one of claims 1 - 6, wherein a plurality of pairs of antibody-like ligand preparations are used in steps e) - k), each of said pairs being used for detecting one particular cell type of interest out of the plurality of particular cell types, each pair comprising
a certain primary antibody-like ligand preparation comprising a ligand capable of specifically binding to a unique cell marker associated with a particular cell type of interest; and
a certain secondary antibody-like ligand preparation comprising a ligand capable of specifically binding to said certain primary antibody-like ligand, which secondary antibody-like ligand is covalently bound to a fluorochrome capable of sending out a unique fluorescence signal.

8. A visualization method according to any of claims 1 - 7, wherein the detection enzyme is chosen from the group of alkaline phosphatase and a peroxidase, such as horseradish peroxidase.

9. A visualization method according to claim 8, wherein the substrate(s) added in step d) is chosen from the group of 3,3'-diaminobenzidine (DAB), Betazoid DAB, Deep Space Black, Bajoran Purple, Warp Red, Ferangi Blue, Vulcan Fast Red, and Vina green.

10. A visualization method according to any one of claims 1 - 9, wherein the fluorochrome(s) are selected from the group of the fluorochromes of the group of Alexa Fluor fluorochromes e.g. Alexa Fluor 680, Alexa Fluor 488, Alexa Fluor 350 and Alexa Fluor 555; the Cy fluorochromes such as Cy2, and Cy7; the DyLight fluorochromes e.g. DyLight 350, DyLight 488, DyLight 555, DyLight750; TRITC, FITC, Texas Red.

11. A visualization method according to any of claims 1 - 10, wherein the visualization step h) is carried out by obtaining one or more photographic images of the sample.

12. A visualization method according to claim 11, wherein the obtained images are digitalized according to known principles and combined into a composite image.

13. A visualization method according to any of claims 1 - 12, wherein at least 4 different cell markers or non-cellular structures including markers representing confounding cells and particular cell types are detected, and preferably at least 5 different cell markers are detected.

## Patentansprüche

1. Verfahren zur Visualisierung einer Vielzahl von Zelltypen in einer mikroskopischen vielzelligen Gewebeprobe, die Zellen mindestens eines bestimmten Zelltyps sowie Zellen mindestens eines störenden Zelltyps umfasst, wobei Zellen des mindestens einen bestimmten Zelltyps und Zellen des mindestens einen störenden Zelltyps beide einen zweiten Zellmarker umfassen und Zellen des mindestens einen störenden Zelltyps auch einen ersten Zellmarker umfassen, der in Zellen des mindestens einen bestimmten Zelltyps nicht vorhanden ist, umfassend die folgenden Schritte:
a) Bereitstellen einer mikroskopischen vielzelligen Gewebeprobe, von der bekannt ist, dass sie Zellen mindestens eines störenden Zelltyps sowie Zellen mindestens eines bestimmten Zelltyps umfasst;
b) Inkubieren der mikroskopischen vielzelligen Gewebeprobe aus Schritt a) mit einem ersten Cocktail aus mindestens einem antikörperartigen Liganden, wobei der bzw. die antikörperartigen Liganden des Cocktails spezifisch an den bzw. die ersten Zellmarker auf störenden Zellen, aber nicht an die zweiten Zellmarker auf Zellen des mindestens einen bestimmten Zelltyps binden kann bzw. können und wobei die Probe mit dem ersten Cocktail unter Bedingungen inkubiert wird, die für die Bildung spezifischer Bindungen zwischen den ersten Zellmarkern auf störenden Zellen und den Liganden günstig sind, was zur Bildung von Komplexen zwischen den ersten Zellmarkern der Zellen und den Liganden führt;
c) Inkubieren der in Schritt b) gebildeten Komplexe mit einer ersten detektionsantikörperartigen Ligandenpräparation, wobei der erste detektionsantikörperartige Ligand an ein erstes Detektionsenzym konjugiert ist, wobei der erste detektionsantikörperartige Ligand spezifisch an die antikörperartigen Liganden des ersten Cocktails binden kann, wobei die Inkubation unter Bedingungen durchgeführt wird, die für die Bildung spezifischer Bindungen zwischen dem ersten detektionsantikörperartigen Liganden und dem bzw. den antikörperartigen Liganden des ersten Cocktails günstig sind, was zur Bildung von Komplexen zwischen dem ersten detektionsantikörperartigen Liganden und den in Schritt b) gebildeten Komplexen führt;
d) Zugeben einer ersten Substratpräparation, umfassend ein Substrat für das erste Detektionsenzym, zu dem in Schritt c) gebildeten Komplex und Inkubieren der resultierenden Mischung unter Bedingungen, die für die Bildung eines detektierbaren blockierenden Polymers günstig sind, wodurch detektierbare angesammelte blockierende Polymere innerhalb oder in der Nähe von störenden Zellen der mikroskopischen vielzelligen Gewebeprobe erhalten werden, wobei das blockierende Polymer den Zugang zu dem zweiten Zellmarker der störenden Zellen verhindert;
e) Zugeben einer ersten primärantikörperartigen Ligandenpräparation, wobei der Ligand spezifisch an den zweiten Zellmarker auf dem mindestens einen bestimmten Zelltyp binden kann, und Inkubieren der resultierenden Mischung unter Bedingungen, die für die Bildung von Komplexen zwischen dem zweiten Zellmarker und dem ersten primärantikörperartigen Liganden günstig sind;
f) Zugeben einer ersten sekundärantikörperartigen Ligandenpräparation zu der resultierenden Mischung von Schritt e), wobei der erste sekundärantikörperartige Ligand an ein Fluorochrom konjugiert ist, wobei der erste sekundärantikörperartige Ligand spezifisch an den ersten primärantikörperartigen Liganden binden kann, und Inkubieren der resultierenden Mischung unter Bedingungen, die für die Bildung spezifischer Bindungen zwischen dem ersten primär- und dem ersten sekundärantikörperartigen Liganden günstig sind;
g) Entfernen ungebundener sekundärantikörperartiger Liganden, beispielsweise durch einen Waschschritt;
h) chemisch Fixieren von Komplexen gebundener Primär und sekundärantikörperartiger Liganden;
i) Aussetzen von Zellen der resultierenden Probe von Schritt h) einer Gruppe aus mindestens einer fluorochrommarkierten antikörperartigen Ligandenpräparation, wobei jede Präparation der Gruppe aus mindestens einer fluorochrommarkierten antikörperartigen Ligandenpräparation spezifisch an einen einzigartigen weiteren Zellmarker binden kann, der mit mindestens einem bestimmten Zelltyp von Interesse assoziiert ist, wobei jede Präparation der Gruppe aus mindestens einer fluorochrommarkierten antikörperartigen Ligandenpräparation ein einzigartiges Fluorochrom aufweist, das ein einzigartiges Fluoreszenzsignal aussenden kann;
j) Entfernen ungebundener fluorochrommarkierter Liganden, beispielsweise durch einen Waschschritt; und
k) Visualisieren des mindestens einen bestimmten Zelltyps und des mindestens einen störenden Zelltyps während des Verlaufs des Verfahrens durch Detektieren von Fluoreszenzsignalen, die von einem oder mehreren Fluorochromen erhalten werden, und durch Detektieren des blockierten Polymers, das innerhalb oder in der Nähe des mindestens einen störenden Zelltyps gebildet worden ist.

2. Visualisierungsverfahren nach Anspruch 1, wobei ein zweiter Cocktail aus mindestens einem antikörperartigen Liganden zusammen mit dem ersten Cocktail in Schritt b) verwendet wird, wobei der bzw. die antikörperartigen Liganden des zweiten Cocktails spezifisch an (einen) andere(n) Zellmarker der störenden Zelltypen binden kann bzw. können als die Liganden des ersten Cocktails, und wobei in Schritt c) eine zweite detektionsantikörperartige Ligandenpräparation verwendet wird, wobei der zweite detektionsantikörperartige Ligand spezifisch an den bzw. die antikörperartigen Liganden des zweiten Cocktails binden kann, wobei der zweite detektionsantikörperartige Ligand ein zweites Detektionsenzym aufweist, und wobei in Schritt d) eine zweite Substratpräparation, die ein Substrat für das zweite Detektionsenzym umfasst, zugegeben wird, was zur Ansammlung eines zusätzlichen blockierenden Polymers innerhalb oder in der Nähe störender Zellen führt, die an den zweiten Cocktail binden, und wobei in Schritt h) die Ansammlung von zusätzlichem blockierendem Polymer detektiert wird.

3. Visualisierungsverfahren nach Anspruch 1, wobei die folgenden Schritte nach Schritt c) und vor Schritt d) durchgeführt werden:
1) Inkubieren der resultierenden Mischung von Schritt c) mit einem zweiten Cocktail aus antikörperartigen Liganden, wobei der bzw. die antikörperartigen Liganden des zweiten Cocktails spezifisch an andere Zellmarker anderer störender Zelltypen binden kann bzw. können als der erste Cocktail, unter Bedingungen, die es Liganden des zweiten Cocktails erlauben, an die anderen Zellmarker zu binden; und
2) Inkubieren der resultierenden Mischung von Schritt 1) mit einem zweiten detektionsantikörperartigen Liganden, wobei der zweite detektionsantikörperartige Ligand spezifisch an den bzw. die antikörperartigen Liganden des zweiten Cocktails binden kann, wobei der zweite detektionsantikörperartige Ligand ein zweites Detektionsenzym aufweist;
und wobei in Schritt d) eine zweite Substratpräparation, die ein Substrat für das zweite Detektionsenzym umfasst, zugegeben wird, was zur Ansammlung eines zusätzlichen blockierenden Polymers innerhalb oder in der Nähe störender Zellen führt, die an den zweiten Cocktail binden, und wobei in Schritt h) die Ansammlung von zusätzlichem blockierendem Polymer detektiert wird.

4. Visualisierungsverfahren nach Anspruch 3, wobei die Schritte 1) und 2) mit einem dritten Cocktail aus antikörperartigen Liganden, einem dritten detektionsantikörperartigen Liganden und einer dritten Substratpräparation, die ein Substrat für das dritte Detektionsenzym umfasst, wiederholt werden, wobei der bzw. die antikörperartigen Liganden des dritten Cocktails spezifisch an andere erste Zellmarker anderer störender Zelltypen binden kann bzw. können als der erste und der zweite Cocktail, wobei der dritte detektionsantikörperartige Ligand spezifisch an den bzw. die antikörperartigen Liganden des dritten Cocktails binden kann, wobei der dritte detektionsantikörperartige Ligand ein drittes Detektionsenzym aufweist, wobei die dritte Substratpräparation ein Substrat für das dritte Detektionsenzym umfasst, und wobei in Schritt h) die Ansammlung von zusätzlichem blockierenden Polymer detektiert wird.

5. Visualisierungsverfahren nach Anspruch 4, wobei die Schritte 1) und 2) mit einem vierten Cocktail aus antikörperartigen Liganden, einem vierten detektionsantikörperartigen Liganden und einer vierten Substratpräparation, die ein Substrat für das vierte Detektionsenzym umfasst, wiederholt werden, wobei der bzw. die antikörperartigen Liganden des vierten Cocktails spezifisch an andere erste Zellmarker anderer störender Zelltypen binden kann bzw. können als der erste, zweite und dritte Cocktail, wobei der vierte detektionsantikörperartige Ligand spezifisch an den bzw. die antikörperartigen Liganden des vierten Cocktails binden kann, wobei der vierte detektionsantikörperartige Ligand ein viertes Detektionsenzym aufweist, wobei die vierte Substratpräparation ein Substrat für das vierte Detektionsenzym umfasst, und wobei in Schritt h) die Ansammlung von zusätzlichem blockierenden Polymer detektiert wird.

6. Visualisierungsverfahren nach einem der Ansprüche 3 bis 5, wobei die Farbe des aus der ersten Substratpräparation gebildeten angesammelten blockierenden Polymers dunkel und/oder opak ist und dass die Farben der anschließend gebildeten angesammelten blockierenden Polymere stufenweise heller und/oder weniger opak werden.

7. Visualisierungsverfahren nach einem der Ansprüche 1 bis 6, wobei in den Schritten e) bis k) eine Vielzahl von Paaren von antikörperartigen Ligandenpräparationen verwendet werden, wobei jedes der Paare zum Detektieren eines bestimmten Zelltyps von Interesse aus der Vielzahl von bestimmten Zelltypen verwendet wird, wobei jedes Paar Folgendes umfasst:
eine bestimmte primärantikörperartige Ligandenpräparation, umfassend einen Liganden, der spezifisch an einen einzigartigen Zellmarker binden kann, der mit einem bestimmten Zelltyp von Interesse assoziiert ist;
eine bestimmte sekundärantikörperartige Ligandenpräparation, umfassend einen Liganden, der spezifisch an den bestimmten primärantikörperartigen Liganden binden kann, wobei der sekundärantikörperartige Ligand kovalent an ein Fluorochrom gebunden ist, das ein einzigartiges Fluoreszenzsignal aussenden kann.

8. Visualisierungsverfahren nach einem der Ansprüche 1 bis 7, wobei das Detektionsenzym aus der Gruppe aus alkalischer Phosphatase und einer Peroxidase, wie beispielsweise Meerrettichperoxidase, ausgewählt ist.

9. Visualisierungsverfahren nach Anspruch 8, wobei das bzw. die in Schritt d) zugegebenen Substrate aus der Gruppe aus 3,3'-Diaminobenzidin (DAB), Betazoid-DAB, Deep Space Black, Bajoran Purple, Warp Red, Ferangi Blue, Vulcan Fast Red und Vina-Grün ausgewählt ist bzw. sind.

10. Visualisierungsverfahren nach einem der Ansprüche 1 bis 9, wobei das bzw. die Fluorochrome aus der Gruppe der Fluorochrome der Gruppe von Alexa-Fluor-Fluorochromen, z. B. Alexa Fluor 680, Alexa Fluor 488, Alexa Fluor 350 und Alexa Fluor 555; der Cy-Fluorochrome wie Cy2 und Cy7; der DyLight-Fluorochrome, z. B. DyLight 350, DyLight 488, DyLight 555, DyLight 750; TRITC, FITC, Texas Rot ausgewählt ist bzw. sind.

11. Visualisierungsverfahren nach einem der Ansprüche 1 bis 10, wobei der Visualisierungsschritt h) durchgeführt wird, indem eines oder mehrere photographische Bilder der Probe erhalten werden.

12. Visualisierungsverfahren nach Anspruch 11, wobei die erhaltenen Bilder nach bekannten Prinzipien digitalisiert und zu einem zusammengesetzten Bild kombiniert werden.

13. Visualisierungsverfahren nach einem der Ansprüche 1 bis 12, wobei mindestens 4 verschiedene Zellmarker oder nicht-zelluläre Strukturen einschließlich Markern, die störende Zellen und bestimmte Zelltypen darstellen, detektiert werden und vorzugsweise mindestens 5 verschiedene Zellmarker detektiert werden.

## Revendications

1. Procédé de visualisation d'une pluralité de types de cellules dans un échantillon de tissu à cellules multiples microscopiques comprenant des cellules d'au moins un type de cellules particulières ainsi que des cellules d'au moins un type de cellules combinées, dans lequel les cellules dudit au moins un type de cellules particulières et les cellules dudit au moins un type de cellules combinées comprennent toutes un deuxième marqueur de cellules et les cellules de l'au moins un type de cellules combinées comprennent également un premier marqueur de cellules absent des cellules de l'au moins un type de cellules particulières, comprenant les étapes de :
a) fourniture d'un échantillon de tissu à cellules multiples microscopiques dont on sait qu'il comprend des cellules d'au moins un type de cellules combinées ainsi que des cellules d'au moins un type de cellules particulières ;
b) incubation de l'échantillon de tissu à cellules multiples microscopiques de l'étape a) avec un premier cocktail d'au moins un ligand de type anticorps, lequel (lesquels) ligand(s) de type anticorps du cocktail est (sont) capable(s) de se lier spécifiquement au dit (auxdits) premier(s) marqueur(s) de cellules sur les cellules combinées mais pas auxdits deuxièmes marqueurs de cellules sur les cellules dudit au moins un type de cellules particulières, et où l'échantillon est incubé avec le premier cocktail dans des conditions favorables à la formation de liaisons spécifiques entre lesdits premiers marqueurs de cellules sur les cellules combinées et lesdits ligands, résultant en la formation de complexes entre lesdits premiers marqueurs de cellules desdites cellules et lesdits ligands ;
c) incubation des complexes formés à l'étape b) avec une première préparation de ligand de type anticorps de détection, le premier ligand de type anticorps de détection étant conjugué à une première enzyme de détection, ledit premier ligand de type anticorps de détection étant capable de se lier spécifiquement aux ligands de type anticorps dudit premier cocktail, où l'incubation est réalisée dans des conditions favorables à la formation de liaisons spécifiques entre ledit premier ligand de type anticorps de détection et le (les) ligand(s) de type anticorps dudit premier cocktail, résultant en la formation de complexes entre ledit premier ligand de type anticorps de détection et les complexes formés à l'étape b) ;
d) ajout d'une première préparation de substrat comprenant un substrat pour ladite première enzyme de détection au complexe formé à l'étape c) et incubation du mélange résultant dans des conditions qui sont favorables à la formation d'un polymère de blocage détectable permettant ainsi d'obtenir des polymères de blocage accumulés à l'intérieur ou autour des cellules combinées dudit échantillon de tissu à cellules multiples microscopiques, lequel polymère de blocage empêche l'accès au deuxième marqueur de cellules des cellules combinées ;
e) l'ajout d'une première préparation de ligand de type anticorps primaire, dans lequel le ligand est capable de se lier spécifiquement au deuxième marqueur de cellules sur ledit au moins un type de cellules particulières, et l'incubation du mélange résultant dans des conditions favorables à la formation de complexes entre ledit marqueur de cellules et ledit premier ligand de type anticorps primaire ;
f) l'ajout d'une première préparation de ligand de type anticorps secondaire au mélange résultant de l'étape e), le premier ligand de type anticorps secondaire étant conjugué à un fluorochrome, ledit premier ligand de type anticorps secondaire étant capable de se lier spécifiquement au dit premier ligand de type anticorps primaire, et l'incubation du mélange résultant dans des conditions favorables à la formation de liaisons spécifiques entre lesdits premiers ligands de type anticorps primaire et secondaire ;
g) l'élimination des ligands de type anticorps secondaire non liés, par exemple par une étape de lavage :
h) la fixation chimique des complexes des ligands de type anticorps primaire et secondaire liés ;
i) l'exposition des cellules de l'échantillon résultant de l'étape h) à un groupe d'au moins une préparation de ligand de type anticorps marqué par un fluorochrome, chaque préparation dudit groupe d'au moins une préparation de ligand de type anticorps marqué par un fluorochrome étant capable de se lier spécifiquement à un autre marqueur de cellules unique associé à au moins un type de cellules particulières d'intérêt, chaque préparation dudit groupe d'au moins une préparation de ligand de type anticorps marqué par un fluorochrome ayant un fluorochrome unique capable d'envoyer un signal de fluorescence unique ;
j) l'élimination des ligands marqués par un fluorochrome non lié, par exemple par une étape de lavage ; et
k) au cours du procédé, la visualisation dudit au moins un type de cellules particulières et dudit au moins un type de cellules combinées par détection des signaux de fluorescence obtenus d'un fluorochrome ou plus, et par détection du polymère de blocage qui a été formé à l'intérieur ou autour dudit au moins un type de cellules combinées.

2. Procédé de visualisation selon la revendication 1, dans lequel un deuxième cocktail d'au moins un ligand de type anticorps est utilisé conjointement au premier cocktail à l'étape b), dans lequel le ou les ligand(s) de type anticorps du deuxième cocktail sont capables de se lier spécifiquement à un autre (d'autres) marqueurs de cellules des types de cellules combinées par rapport aux ligands dudit premier cocktail et dans lequel une deuxième préparation de ligand de type anticorps de détection est utilisée à l'étape c), le deuxième ligand de type anticorps de détection étant capable de se lier spécifiquement au ligand (aux ligands) de type anticorps du deuxième cocktail, ledit deuxième ligand de type anticorps de détection ayant une deuxième enzyme de détection, et dans lequel une deuxième préparation de substrat comprenant un substrat pour ladite deuxième enzyme de détection est ajoutée à l'étape d) résultant en l'accumulation d'un polymère de blocage, supplémentaire à l'intérieur ou autour des cellules combinées se liant au deuxième cocktail, et où ladite accumulation du polymère de blocage supplémentaire est détectée à l'étape h).

3. Procédé de visualisation selon la revendication 1, dans lequel les étapes suivantes sont réalisées après l'étape c) et avant l'étape d) :
1) incubation du mélange résultant de l'étape c) avec un deuxième cocktail de ligands de type anticorps, dans lequel le (les) ligands de type anticorps du deuxième cocktail est/sont capables de se lier spécifiquement à un autre (d'autres) marqueur(s) de cellules d'un autre type de cellules combinées par rapport au dit premier cocktail, dans des conditions permettant aux ligands du deuxième cocktail de se lier auxdits autres marqueurs de cellules ; et
2) incubation du mélange résultant de l'étape 1) avec un deuxième ligand de type anticorps de détection, le deuxième ligand de type anticorps de détection étant capable de se lier spécifiquement au ligand (aux ligands) de type anticorps du deuxième cocktail, ledit deuxième ligand de type anticorps de détection ayant une deuxième enzyme de détection ;
et dans lequel une deuxième préparation de substrat comprenant un substrat pour ladite deuxième enzyme de détection est ajoutée à l'étape d) résultant en l'accumulation d'un polymère de blocage supplémentaire à l'intérieur ou autour des cellules combinées se liant au deuxième cocktail, et où ladite accumulation du polymère de blocage supplémentaire est détectée à l'étape h).

4. Procédé de visualisation selon la revendication 3, dans lequel les étapes 1) et 2) sont répétées avec un troisième cocktail de ligands de type anticorps, un troisième ligand de type anticorps de détection, et une troisième préparation de substrat comprenant un substrat pour ladite troisième enzyme de détection, ledit (lesdits) ligands de type anticorps du troisième cocktail est/sont capables de se lier spécifiquement à un autre (d'autres) premier(s) marqueur(s) de cellules d'un autre type de cellules combinées par rapport auxdits premier et deuxième cocktails, ledit troisième ligand de type anticorps de détection étant capable de se lier spécifiquement au ligand (aux ligands) de type anticorps du troisième cocktail, ledit troisième ligand de type anticorps de détection ayant une troisième enzyme de détection, ladite troisième préparation de substrat comprenant un substrat pour ladite troisième enzyme de détection, et où ladite accumulation du polymère de blocage supplémentaire est détectée à l'étape h).

5. Procédé de visualisation selon la revendication 4, dans lequel les étapes 1) et 2) sont répétées avec un quatrième cocktail de ligands de type anticorps, un quatrième ligand de type anticorps de détection, et une quatrième préparation de substrat comprenant un substrat pour ladite quatrième enzyme de détection, ledit (lesdits) ligand(s) de type anticorps du quatrième cocktail est/sont capables de se lier spécifiquement à un autre (d'autres) premier marqueur(s) de cellules d'un autre type de cellules combinées par rapport auxdits premier, deuxième et troisième cocktails, ledit quatrième ligand de type anticorps de détection étant capable de se lier spécifiquement au ligand (aux ligands) de type anticorps dudit quatrième cocktail, ledit quatrième ligand de type anticorps de détection ayant une quatrième enzyme de détection, ladite quatrième préparation de substrat comprenant un substrat pour ladite quatrième enzyme de détection, et où ladite accumulation du polymère de blocage supplémentaire est détectée à l'étape h).

6. Procédé de visualisation selon l'une quelconque des revendications 3 à 5, dans lequel la couleur du polymère de blocage accumulé formé sur la première préparation de substrat est sombre et/ou opaque et les couleurs des polymères de blocage accumulés formés ensuite sont de plus en plus lumineuses et/ou de moins en moins opaques.

7. Procédé de visualisation selon l'une quelconque des revendications 1 à 6, dans lequel une pluralité de paires de préparations de ligand de type anticorps sont utilisées dans les étapes e) à k), chacune desdites paires étant utilisées pour détecter un type de cellules particulières d'intérêt parmi la pluralité de types de cellules particulières, chaque paire comprenant
une certaine préparation de ligand de type anticorps primaire comprenant un ligand capable de se lier spécifiquement à un marqueur de cellules unique associé à un type de cellules particulières d'intérêt ; et
une certaine préparation de ligand de type anticorps secondaire comprenant un ligand capable de se lier spécifiquement à un certain ligand de type anticorps primaire, lequel ligand de type anticorps secondaire est lié de façon covalente à un fluorochrome capable d'envoyer un signal de fluorescence unique.

8. Procédé de visualisation selon l'une quelconque des revendications 1 à 7, dans lequel l'enzyme de détection est choisie dans le groupe de la phosphatase alcaline et d'une peroxydase, telle que la peroxydase de raifort.

9. Procédé de visualisation selon la revendication 8, dans lequel le(s) substrat(s) ajouté(s) à l'étape d) est (sont) choisi(s) dans le groupe comprenant les 3,3'-diaminobenzidine (DAB), Betazoid DAB, Deep Space Black, Bajoran Purple, Warp Red, Ferangi Blue, Vulcan Fast Red, et Vina green.

10. Procédé de visualisation selon l'une quelconque des revendications 1 à 9, dans lequel le(s) fluorochrome(s) sont sélectionnés dans le groupe des fluorochromes du groupe des fluorochromes Alexa Fluor, par exemple, Alexa Fluor 680, Alexa Fluor 488, Alexa Fluor 350 et Alexa Fluor 555 ; des fluorochromes Cy tels que Cy2, Cy7, et Cy7 ; des fluorochromes DyLight par exemple, DyLight 350, DyLight 488, DyLight 555, DyLight750 ; TRITC, FITC, Texas Red.

11. Procédé de visualisation selon l'une quelconque des revendications 1 à 10, dans lequel l'étape de visualisation h) est réalisée par obtention d'une ou plusieurs images photographiques de l'échantillon.

12. Procédé de visualisation selon la revendication 11, dans lequel les images obtenues sont numérisées selon des principes connus et combinées en une image composite.

13. Procédé de visualisation selon l'une quelconque des revendications 1 à 12, dans lequel au moins 4 marqueurs de cellules différents ou marqueurs comprenant des structures non cellulaires représentant des types de cellules combinées ou de cellules particulières sont détectés, et de préférence au moins 5 marqueurs de cellules différents sont détectés.
